# DEMANDE DE BREVET EUROPEEN

(11) **EP 4 803 052 A1**
(43) Date de publication de la demande: **09.09.2026**
(21) Numéro de dépôt: 26161752.6
(22) Date de dépôt: 02.03.2026
(51) Int. Cl.: A61F 2/58, A61F 2/50, A61F 2/68

(54) **SYSTÈME HAPTIQUE POUR UNE PROTHÈSE**

(30) Priorité: 03.03.2025 FR 2502140
(71) Demandeur: Advanced Care Technologies, 78790 Courgent (FR)
(72) Inventeur: ELEUTERIO, Gabriel, 78790 COURGENT (FR)
(74) Mandataire: Regimbeau

(57) **Abrégé**

Le présent exposé concerne un système haptique (3) pour une prothèse (1) fixée à un moignon (2), le système comprenant :
un capteur ;
un manchon (81) ; et
un stimulateur.

## Description

### DOMAINE TECHNIQUE

Le présent exposé concerne le domaine des prothèses médicales. Plus précisément, le présent exposé concerne la simulation sensorielle à l'aide d'un système haptique pour une prothèse fixée à un moignon.

### ETAT DE LA TECHNIQUE

Un système haptique pour simuler un retour sensoriel à une personne amputée comprend généralement des capteurs, positionnés sur la prothèse, qui communiquent avec un stimulateur positionné sur le moignon.

La fixation des capteurs à la prothèse présente toutefois des difficultés. En effet, la présence du capteur ne doit pas gêner la préhension, tout en en restituant les effets de manière suffisamment précise. En outre, un même système doit pouvoir être réutilisé sur des prothèses différentes et dans des conditions d'utilisation différentes.

Par ailleurs, les fils d'alimentation électrique des capteurs peuvent être source d'un encombrement important.

### EXPOSE GENERAL

Un objectif du présent exposé est de fixer un capteur d'un système haptique à une prothèse de manière simple, pratique, et peu coûteuse.

Un autre objectif du présent exposé est de réduire l'encombrement d'un système haptique pour une prothèse.

A cet égard, il est proposé, selon un premier aspect du présent exposé, un système haptique pour une prothèse fixée à un moignon, le système comprenant :
un capteur ;
un manchon configuré pour être porté sur un doigt de la prothèse et comprenant :
   une première couche destinée à être au contact du doigt lorsque le manchon est porté sur le doigt, la première couche étant formée en un premier matériau présentant une première dureté Shore ;
   une deuxième couche en contact avec la première couche, le capteur étant noyé dans la deuxième couche, la deuxième couche étant formée en un deuxième matériau, distinct du premier matériau, et présentant une deuxième dureté Shore strictement supérieure à la première dureté Shore ; et
   une troisième couche en contact avec la deuxième couche de sorte que la deuxième couche s'étend entre la première couche et la troisième couche, la troisième couche étant formée en un troisième matériau, distinct du premier matériau et du deuxième matériau, et présentant une troisième dureté Shore strictement supérieure à la deuxième dureté Shore ; et
un stimulateur configuré pour être fixé sur le moignon et pour stimuler le moignon à partir d'une sollicitation exercée sur la prothèse et mesurée par le capteur.

Selon le premier aspect du présent exposé, le système haptique peut en outre comprendre :
un support configuré pour être fixé à la prothèse de manière amovible ; et
un organe de contrôle fixé sur le support, et configuré pour être relié via un fil électrique au capteur pour en recevoir un signal représentatif d'une mesure de la sollicitation exercée sur la prothèse.

Selon un deuxième aspect du présent exposé, il est proposé un système haptique pour une prothèse fixée à un moignon, le système comprenant :
un capteur ;
un support configuré pour être fixé à la prothèse de manière amovible ;
un organe de contrôle fixé sur le support, et configuré pour être relié via un fil électrique au capteur pour en recevoir un signal représentatif d'une mesure d'une sollicitation exercée sur la prothèse et mesurée par le capteur ; et
un stimulateur configuré pour être fixé sur le moignon et pour stimuler le moignon à partir de la sollicitation exercée sur la prothèse et mesurée par le capteur.

Selon le deuxième aspect du présent exposé, le système peut en outre comprendre un manchon configuré pour être porté sur un doigt de la prothèse et comprenant :
une première couche destinée à être au contact du doigt lorsque le manchon est porté sur le doigt, la première couche étant formée en un premier matériau présentant une première dureté Shore ;
une deuxième couche en contact avec la première couche, le capteur étant noyé dans la deuxième couche, la deuxième couche étant formée en un deuxième matériau, distinct du premier matériau, et présentant une deuxième dureté Shore strictement supérieure à la première dureté Shore ; et
une troisième couche en contact avec la deuxième couche de sorte que la deuxième couche s'étend entre la première couche et la troisième couche, la troisième couche étant formée en un troisième matériau, distinct du premier matériau et du deuxième matériau, et présentant une troisième dureté Shore strictement supérieure à la deuxième dureté Shore.

Selon l'un du premier aspect et du deuxième aspect du présent exposé, le manchon peut être constitué de la première couche, de la deuxième couche, et de la troisième couche. La première dureté Shore peut être supérieure ou égale à Shore 00-10 et inférieure ou égale à Shore 00-50, la deuxième dureté Shore être supérieure ou égale à Shore 5A et inférieure ou égale à Shore 15A, et/ou la troisième dureté Shore être supérieure ou égale à Shore 25A et inférieure ou égale à Shore 35A. Le premier matériau peut présenter une première élongation à la rupture, le deuxième matériau présenter une deuxième élongation à la rupture, et le troisième matériau présenter une troisième élongation à la rupture, la première élongation à la rupture étant strictement supérieure à la deuxième élongation à la rupture, et la deuxième élongation à la rupture étant strictement supérieure à la troisième élongation à la rupture. La première élongation à la rupture peut être supérieure ou égale à 800 % et inférieure ou égale à 1000 %, la deuxième élongation à la rupture être supérieure ou égale à 550 % et inférieure ou égale à 750 %, et/ou la troisième élongation à la rupture être supérieure ou égale à 250 % et inférieure ou égale à 450 %. La première couche peut présenter une première épaisseur, la deuxième couche présenter une deuxième épaisseur, et la troisième couche présenter une troisième épaisseur, chacune de la première épaisseur, de la deuxième épaisseur, et de la troisième épaisseur, étant supérieure ou égale à 0,7 mm et inférieure ou égale à 1,5 mm. La première épaisseur, la deuxième épaisseur, et la troisième épaisseur peuvent être identiques entre elles. Chacun du premier matériau, du deuxième matériau, et du troisième matériau, peut être un silicone. Le premier matériau peut être obtenu par une première polymérisation mise en œuvre pendant une première durée supérieure ou égale à 3 minutes et inférieure ou égale à 15 minutes, le deuxième matériau être obtenu par une deuxième polymérisation mise en œuvre pendant une deuxième durée supérieure ou égale à 30 minutes et inférieure ou égale à 120 minutes, et le troisième matériau être obtenu par une troisième polymérisation mise en œuvre pendant une troisième durée supérieure ou égale à 600 minutes et inférieure ou égale à 1200 minutes.

### DESCRIPTION DES FIGURES

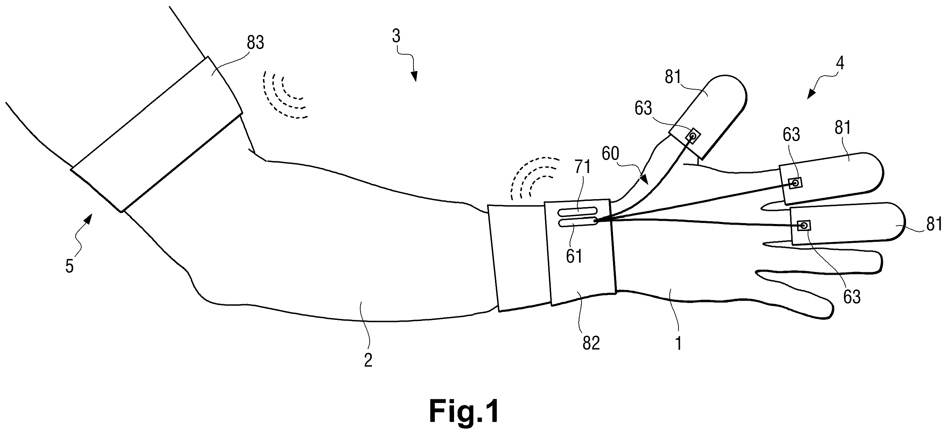

La figure 1 illustre schématiquement un système haptique pour une prothèse fixée sur un moignon.

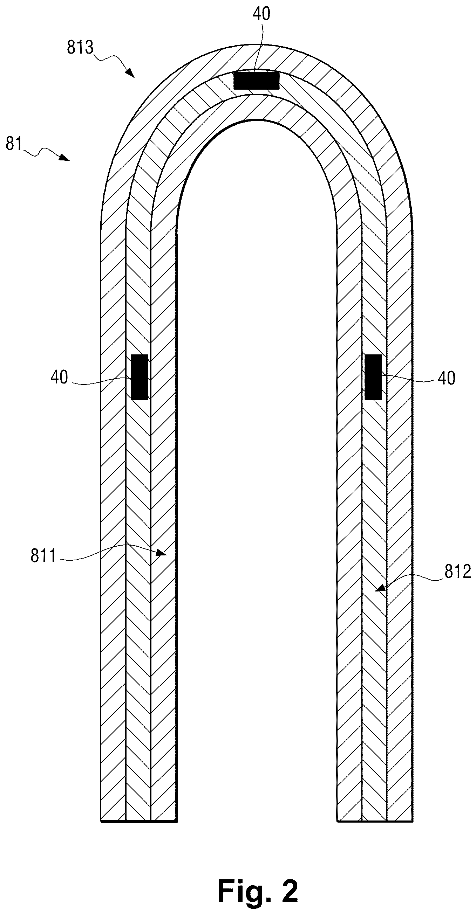

La figure 2 illustre schématiquement un manchon d'un système haptique pour une prothèse fixée sur un moignon.

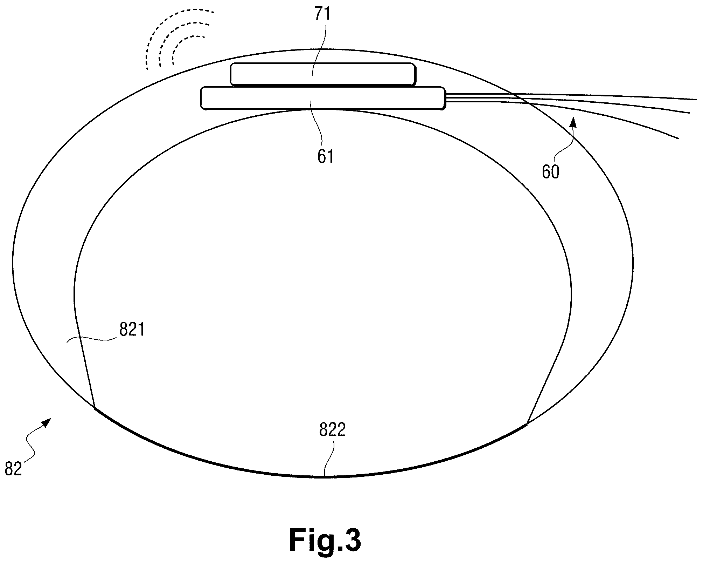

La figure 3 illustre schématiquement un bracelet d'un système haptique pour une prothèse fixée sur un moignon.

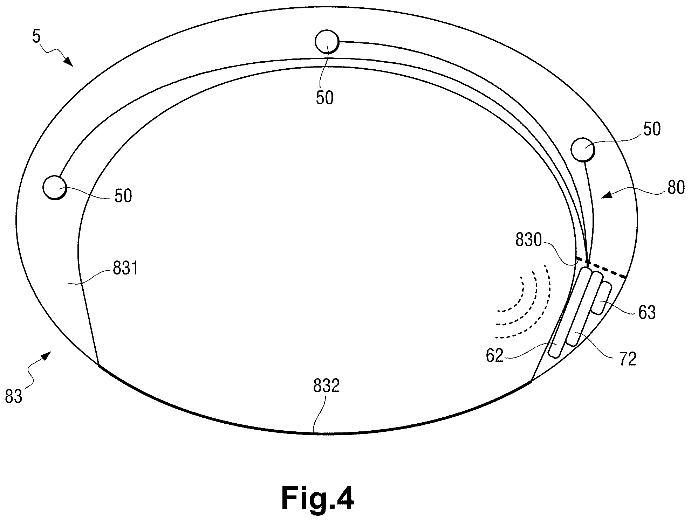

La figure 4 illustre schématiquement un brassard d'un système haptique pour une prothèse fixée sur un moignon.

### DESCRIPTION DETAILLEE

### Prothèse et moignon

Une personne amputée de tout ou partie d'un membre peut être munie d'une prothèse **1** visant à remplacer tout ou partie de la portion de membre amputée.

Le membre peut être un membre supérieur, tel qu'une main, ou un membre inférieur, tel qu'une jambe.

La prothèse **1** est fixée, de manière amovible ou non, à la partie du membre restante, appelée moignon **2.** Une fixation amovible permet de fixer temporairement un élément à un autre, tout en offrant la possibilité de séparer les éléments sans les endommager. La prothèse **1** peut être myoélectrique, c'est-à-dire utiliser des signaux électriques générés par les muscles du moignon **2** pour contrôler ses mouvements. Ceci n'est toutefois pas limitatif, puisque la prothèse **1** peut également être mécanique et/ou esthétique.

### Système haptique

Un système haptique **3** pour une prothèse **1** fixée sur un moignon **2** permet de restituer à son porteur tout ou partie de l'activité sensorielle native de la portion du membre dont il a été amputé.

Le système haptique **3** comprend un dispositif de mesure **4,** un dispositif de stimulation **5,** un dispositif de contrôle **61, 62,** et un dispositif d'alimentation électrique **71, 72.**

Le dispositif de mesure **4** est configuré pour mesurer une sollicitation exercée sur la prothèse **1.** La sollicitation peut être de toute nature, par exemple mécanique, tel qu'un effort, ou thermique, tel qu'un échauffement. Le dispositif de mesure **4** peut être actif, c'est-à-dire nécessiter l'apport d'une énergie externe, typiquement une alimentation électrique, pour mesurer la sollicitation, ou être passif, c'est-à-dire ne pas nécessiter l'apport d'une énergie externe pour mesurer la sollicitation.

Le dispositif de stimulation **5** est configuré pour stimuler le moignon **2** à partir de la sollicitation ainsi mesurée. De cette manière, tout ou partie de l'activité sensorielle de la portion de membre amputée est restituée au porteur de la prothèse **1.** En d'autres termes, le dispositif de stimulation **5** permet un retour haptique au porteur à la suite d'une sollicitation de la prothèse **1.** Le dispositif de stimulation **5** peut stimuler le moignon **2** de différentes manières, typiquement par vibration.

Le dispositif de contrôle **61, 62** est configuré pour contrôler le dispositif de mesure **4** et/ou le dispositif de stimulation **5.**

Le dispositif d'alimentation électrique **71, 72** est configuré pour fournir une alimentation électrique au dispositif de contrôle **61, 62,** et/ou au dispositif de stimulation **5.** Lorsque le dispositif de mesure **4** est actif, le dispositif d'alimentation électrique **71, 72** est configuré pour lui fournir une alimentation électrique.

Chacun du dispositif de mesure **4,** du dispositif d'alimentation électrique **71, 72,** du dispositif de stimulation **5,** et du dispositif de contrôle **61, 62,** est configuré pour être fixé, en tout ou partie, de manière amovible ou non, à la prothèse **1** et/ou au moignon **2.**

De préférence, le dispositif de mesure **4** est configuré pour être fixé, de manière amovible ou non, à la prothèse **1,** et ce afin de favoriser la mesure de la sollicitation exercée sur celle-ci, tandis que le dispositif de stimulation **5** est configuré pour être fixé, de manière amovible ou non, au moignon **2,** et ce afin de favoriser la stimulation de ce-dernier.

Pour faciliter l'agencement du dispositif de mesure **4,** du dispositif d'alimentation électrique **71, 72,** du dispositif de stimulation **5,** et/ou du dispositif de contrôle **61, 62,** le système haptique **3** comprend une pluralité de supports **81, 82, 83.**

Chacun des supports **81, 82, 83** est configuré pour supporter tout ou partie de l'un du dispositif de mesure **4,** du dispositif d'alimentation électrique **71,** 72, du dispositif de stimulation **5,** et du dispositif de contrôle **61, 62,** lesquels peuvent être fixés sur leur support **81, 82, 83** respectif de manière amovible, ou non.

Chacun des supports **81, 82, 83** peut être fixé, de manière amovible ou non, au moignon **2** et/ou à la prothèse **1.** De cette manière, le système haptique **3** peut être adapté à différents membres, et à différents porteurs. En outre, le système haptique **3** peut facilement être déplacé d'un membre à l'autre, et/ou d'un porteur à l'autre. Enfin, le système haptique **3** est, ainsi, rendu non-invasif.

### Premiers supports

Le dispositif de mesure **4** peut comprendre une pluralité de capteurs **40,** typiquement des capteurs de pression.

Par exemple, le capteur **40** comprend un élément électronique qui voit sa résistance électrique varier en fonction d'une contrainte mécanique auquel l'élément électronique est soumis.

A cet égard, le système haptique **3** comprend une pluralité de premiers supports **81,** chacun étant configuré pour supporter un (ou plusieurs) capteur(s) **40.** Plus précisément, chaque capteur **40** est configuré pour être fixé, de manière amovible, ou non, à un premier support **81.**

En outre, chaque premier support **81** est configuré pour être porté sur une première partie de la prothèse **1,** de sorte que le (ou les) capteur(s) **40** mesure(nt) une sollicitation, typiquement une pression, exercé sur la première partie de la prothèse **1.** De cette manière, une interaction entre la première partie de la prothèse **1** et un élément extérieur peut être mesurée par le dispositif de mesure **4.** Typiquement, lorsque le porteur touche une surface ou attrape un objet, le dispositif de mesure **4** permet de détecter cette interaction et d'en déterminer l'intensité.

De préférence, chaque premier support **81** se présente sous la forme d'un manchon **81** configuré pour être porté par une partie oblongue de la prothèse **1,** typiquement un doigt lorsque la prothèse 1 remplace un membre supérieur amputé. Le manchon **81** présente une forme cylindrique ouverte au niveau d'au moins une de ses extrémité axiales afin de pouvoir être emmanché au niveau de la partie oblongue de la prothèse **1.** La forme de manchon **81** confère au premier support **81** une importante praticité d'utilisation car leur manipulation est aisée.

Le manchon **81** comprend, voire est constitué, de trois couches **811, 812, 813.**

Une première couche **811** du manchon **81** est destinée à être en contact avec la partie oblongue de la prothèse **1** lorsque le manchon **81** est porté sur cette-dernière. De préférence, la première couche **811** recouvre toute la partie oblongue.

La première couche **811** est suffisamment flexible pour garantir l'adhérence du manchon **81** à la prothèse **1,** ainsi que son maintien, tout en laissant de la liberté de mouvement à la prothèse **1.**

La première couche **811** présente une première épaisseur, prise dans une direction orthogonale à la surface externe de la partie oblongue avec laquelle le manchon **81** est en contact lorsqu'il est porté sur cette-dernière. De préférence, la première épaisseur est supérieure ou égale à 0,7 mm et inférieure ou égale à 1,5 mm, par exemple égale à 1,0 mm. La première couche **811** est formée en un premier matériau présentant une première dureté Shore, de préférence supérieure ou égale à Shore 00-10 et inférieure ou égale à Shore 00-50, par exemple égale à Shore 00-35.

Le premier matériau présente en outre une première élongation à la rupture, de préférence supérieure ou égale à 800 % et inférieure ou égale à 1000 %, par exemple égale à 900 %. Dans le cadre du présent exposé, l'élongation à la rupture est définie comme dans la norme ASTM D-412, laquelle est notamment utilisée pour déterminer les propriétés de traction des matériaux en caoutchouc et des élastomères. L'élongation à la rupture représente la quantité d'étirement ou de déformation qu'un matériau peut supporter avant de se rompre sous une contrainte de traction. Dans le cadre de la norme ASTM D-412, l'allongement à la rupture est exprimé en pourcentage de la longueur initiale de l'échantillon. Le test consiste à étirer un échantillon à une vitesse contrôlée jusqu'à ce qu'il se rompe, et l'allongement au point de rupture est enregistré.

De préférence, le premier matériau est un silicone qui, du fait des propriétés exposées, est très souple.

Dans une variante, le premier matériau est obtenu par une première polymérisation mise en œuvre pendant une première durée supérieure ou égale à 3 minutes et inférieure ou égale à 15 minutes, par exemple égale à 5 minutes.

Une deuxième couche **812** du manchon **81** est en contact avec la première couche **811** du manchon **81.** De préférence, la deuxième couche **812** recouvre toute la première couche **811,** c'est-à-dire que la deuxième couche **812** présente une extension surfacique interne au moins égale à une extension surfacique externe de la première couche **811.** Dans le cadre du présent exposé, les termes « interne » et « externe » désignent le positionnement d'une portion d'un élément destiné à être porté par la prothèse **1** et/ou le moignon **2,** en prenant la prothèse **1** et/ou le moignon **2** comme référence, une portion interne étant plus proche de la prothèse **1** et/ou du moignon **2** qu'une portion externe.

Au moins un capteur **40,** de préférence quatre capteurs **40,** est (sont) noyé(s) (ou encapsulé(s)) dans la deuxième couche **812.** Dans une variante, les quatre capteurs **40** sont agencés au sein de la deuxième couche **812** de sorte que, lorsque le manchon **81** est porté sur un doigt d'une prothèse **1** du membre supérieur, un premier capteur **40** se trouve en regard de la face pulpaire du doigt, un deuxième capteur **40** se trouve en regard d'une première face du doigt transversale à la face pulpaire, un troisième capteur **40** se trouve en regard d'une deuxième face du doigt transversale à la face pulpaire, et un quatrième capteur **40** se trouve en regard de l'extrémité du doigt opposée au moignon **2.** De cette manière, la surface de mesure est correctement étendue de sorte à fournir une finesse de mesure suffisante.

La deuxième couche **812** permet de protéger le (ou les) capteur(s) **40.** En outre, elle présente suffisamment de souplesse pour maintenir le manchon **81** sur la prothèse **1,** et suffisamment de résistance pour protéger durablement le (ou les) capteur(s) **40,** même en présence de contraintes extérieures importantes.

La deuxième couche **812** présente une deuxième épaisseur, prise dans une direction orthogonale à la surface externe de la première couche **811** avec laquelle la deuxième couche **812** est en contact. De préférence, la deuxième épaisseur est supérieure ou égale à 0,7 mm et inférieure ou égale à 1,5 mm, par exemple égale à 1,0 mm.

La deuxième couche **812** est formée en un deuxième matériau, distinct du premier matériau, et présentant une deuxième dureté Shore, strictement supérieure à la première dureté Shore, de préférence supérieure ou égale à Shore 5A et inférieure ou égale à Shore 15A, par exemple égale à Shore 10A.

Le deuxième matériau présente en outre une deuxième élongation à la rupture, de préférence strictement inférieure à la première élongation à la première élongation à la rupture, avantageusement supérieure ou égale à 550 % et inférieure ou égale à 750 %, par exemple égale à 663 %.

De préférence, le deuxième matériau est un silicone qui, du fait des propriétés exposées, est relativement souple mais plus rigide que le silicone de la première couche **811.**

Dans une variante, le deuxième matériau est obtenu par une deuxième polymérisation mise en œuvre pendant une deuxième durée supérieure ou égale à 30 minutes et inférieure ou égale à 120 minutes, par exemple égale à 75 minutes.

Une troisième couche **813** du manchon **81** est en contact avec la deuxième couche **812** du manchon **81,** de sorte que la deuxième couche **812** s'étend entre la première couche **811** et la troisième couche **813.** De préférence, la troisième couche **813** recouvre toute la deuxième couche **812,** c'est-à-dire que la troisième couche **813** présente une extension surfacique interne au moins égale à l'extension surfacique externe de la deuxième couche **812.** Avantageusement, l'extension surfacique de la première couche **811,** l'extension surfacique de la deuxième couche **812,** et l'extension surfacique de la troisième couche **813,** sont identiques entre elles.

La troisième couche **813** assure une résistance à l'usure, aux frottements et aux cisaillements, lors de l'usage du système haptique **3,** surtout lorsque sa surface externe constitue la surface externe du manchon **81,** tout en maintenant une souplesse suffisante pour adapter le manchon **81** à n'importe quelle prothèse **1.**

La troisième couche **813** présente une troisième épaisseur, prise dans une direction orthogonale à la surface externe de la deuxième couche **812** avec laquelle la troisième couche **813** est en contact. De préférence, la troisième épaisseur est supérieure ou égale à 0,7 mm et inférieure ou égale à 1,5 mm, par exemple égale à 1,0 mm. De préférence, la première épaisseur, la deuxième épaisseur, et la troisième épaisseur, sont identiques entre elles.

La troisième couche **813** est formée en un troisième matériau, distinct du premier matériau et du deuxième matériau, et présentant une troisième dureté Shore, strictement supérieure à la deuxième dureté Shore, de préférence supérieure ou égale à Shore 25A et inférieure ou égale à Shore 35A, par exemple égale à 30A.

Le troisième matériau présente en outre une troisième élongation à la rupture, de préférence strictement inférieure à la deuxième élongation à la rupture, avantageusement supérieure ou égale à supérieure ou égale à 250 % et inférieure ou égale à 450 %, par exemple égale à 364 %.

De préférence, le troisième matériau est un silicone qui, du fait des propriétés exposées, est à la fois extensible et résistant.

Dans une variante, le troisième matériau est obtenu par une troisième polymérisation mise en œuvre pendant une troisième durée supérieure ou égale à 600 minutes et inférieure ou égale à 1200 minutes, par exemple égale à 960 minutes.

Grâce aux propriétés des trois couches **811, 812, 813,** une bonne répartition des contraintes au sein du manchon **81** est obtenue, ce qui permet d'assurer une bonne résistance globale du manchon **81** tout en maintenant la souplesse nécessaire à l'adaptation du manchon **81** à différentes prothèses **1.** En outre, le manchon **81** permet de réaliser des mesures fiables tout au long de l'utilisation du système haptique **3,** quelles que soient les contraintes extérieures. Les temps de polymérisation des matériaux des différentes couches **811, 812, 813** permettent une fabrication rapide et peu coûteuse du manchon **81.**

### Deuxième support

Le dispositif de contrôle **61, 62** peut comprendre un premier organe de contrôle **61** du dispositif de mesure **4,** et le dispositif d'alimentation électrique **71, 72** comprendre un premier organe d'alimentation électrique **71** du premier organe de contrôle **61.** Lorsque le dispositif de mesure **4** est actif, le premier organe d'alimentation électrique **71** alimente également le dispositif de mesure **4.**

Le système haptique **3** comprend un deuxième support **82** configuré pour être porté sur une deuxième partie de la prothèse **1,** distincte des premières parties de la prothèse **1,** typiquement le poignet dans le cas d'une prothèse **1** d'un membre supérieur. La position du deuxième support **82** sur la prothèse **1** est variable en fonction du niveau d'amputation. Le cas échéant, le deuxième support **82** peut être configuré pour être porté sur le moignon **2,** au niveau d'une extrémité distale de ce-dernier, particulièrement dans les cas d'amputation courte.

Le deuxième support **82** est en outre configuré pour supporter le premier organe de contrôle **61** et le premier organe d'alimentation électrique **71.** Plus précisément, le premier organe de contrôle **61** et le premier organe d'alimentation électrique **71** sont, chacun, configuré pour être fixé, de manière amovible, ou non, au deuxième support **82.**

Le deuxième support **82** peut prendre la forme d'un bracelet **82** comprenant une première portion **821,** formée en un textile extensible, sur lequel le premier organe de contrôle **61** et le premier organe d'alimentation électrique **71** sont fixés. A cet égard, des liants textiles présentant une certaine élasticité peuvent être utilisés, afin de réduire le volume de cette partie du système haptique **3,** ce qui facilite l'emmanchement du bracelet **82** sur la prothèse **1.** En tout état de cause, le matériau textile de la première portion **821** peut présenter une élasticité et une robustesse suffisantes pour permettre un port de longue durée sur la prothèse **1.** Le bracelet **82** peut comprendre une deuxième portion **822** constituée d'un système de maintien adaptable, telle qu'une bande élastique, reliant entre elles les deux extrémité circonférentielles de la première portion **821,** la bande élastique étant destiné à être positionnée au niveau de la face interne de la prothèse 1 ou, le cas échéant, du moignon **2,** lorsque le bracelet **82** est porté, afin de minimiser les frottements avec le reste du corps, typiquement entre le bras et le buste du porteur, mais aussi afin d'ajuster aisément le bracelet **82** sur la prothèse **1.**

Le premier organe de contrôle **61** est configuré pour communiquer avec le dispositif de mesure **4,** de préférence avec chacun des capteurs **40,** avantageusement par voie filaire **60,** de sorte à en recevoir les signaux associés à leur mesure de la sollicitation exercée sur la prothèse **1.** En outre, lorsque les capteurs **40** sont actifs, le premier organe d'alimentation électrique **71** est configuré pour fournir une énergie électrique à chacun des capteurs d'effort **40,** de préférence par voie filaire **60,** avantageusement au moyen de la même voie filaire **60** que celle permettant au premier organe de contrôle **61** de communiquer avec les capteurs d'effort **40.** Le cas échéant, les capteurs d'effort **40** sont reliés au premier organe de contrôle **61,** lequel est relié au premier organe d'alimentation électrique **71,** l'énergie électrique issue de ce-dernier à destination des capteurs d'effort **40** transitant via le premier organe de contrôle **61.** Une voie filaire désigne, dans le cadre du présent exposé, un ensemble comprenant au moins un fil électrique. Dans une variante, un connecteur magnétique **63** peut être fixé, de manière amovible ou non, sur chacun des premiers supports **81,** typiquement au niveau d'une surface externe de la troisième couche **813** du manchon **81.** Le connecteur magnétique **63** est en communication, de préférence par contact électrique, avec le (ou les) capteur(s) **40** fixé au premier support **81.** De cette manière, un fil de communication et/ou d'alimentation électrique entre un premier support **81** et le deuxième support **82** peut présenter une extrémité reliée au deuxième support **82,** et une extrémité configurée pour être reliée de manière amovible au connecteur magnétique **63.** Ainsi, le premier support **81** peut être retiré de la prothèse **1** sans avoir à retirer en même temps le deuxième support **82.**

Prévoir que le premier organe d'alimentation électrique **71** et le premier organe de contrôle **61** sont positionnés au niveau de la prothèse **1,** et non du moignon **2,** réduit l'encombrement du système haptique **3,** par réduction de la longueur du dispositif filaire **60** reliant le premier organe de contrôle **61** et/ou le premier organe d'alimentation électrique **71,** au dispositif de mesure **4.**

### Troisième support

Le dispositif de contrôle **61, 62** peut comprendre un deuxième organe de contrôle **62** du dispositif de stimulation **5,** et le dispositif d'alimentation électrique **71, 72** comprendre un deuxième organe d'alimentation électrique **72** du deuxième organe de contrôle **62** et/ou du dispositif de stimulation **5.**

Le deuxième organe d'alimentation électrique **72** peut être une batterie rechargeable au moyen d'un chargeur magnétique **63.**

Le système haptique **3** comprend un troisième support **83** configuré pour être porté sur le moignon **2.**

Le troisième support **83** est en outre configuré pour supporter le deuxième organe de contrôle **62,** le deuxième organe d'alimentation électrique **72,** le dispositif de stimulation **5** et, le cas échéant, le chargeur magnétique **63.** Plus précisément, le deuxième organe d'alimentation électrique **72,** le dispositif de stimulation **5** et, le cas échéant, le chargeur magnétique **63,** sont, chacun, configuré pour être fixé, de manière amovible, ou non, au troisième support **83.**

Le troisième support **83** peut prendre la forme d'un brassard **83** comprenant une première portion **831,** formée en un matériau textile, sur lequel le deuxième organe de contrôle **62,** le deuxième organe d'alimentation électrique **72,** le dispositif de stimulation **5** et, le cas échéant, le chargeur magnétique **63,** sont fixés. A cet égard, des liants textiles présentant une certaine élasticité peuvent être utilisés, afin de réduire le volume de cette partie du système haptique **3,** ce qui non seulement facilite l'emmanchement du brassard **83** sur le moignon **2,** mais améliore en outre la sensation du porteur. En tout état de cause, le matériau textile de la première portion **831** peut présenter une élasticité, une légèreté et une respirabilité suffisantes pour permettre un port de longue durée sur le moignon **2.**

Dans une variante, une couture de séparation **830** peut être prévue au sein de la première portion **831** du brassard **83** pour séparer le deuxième organe de contrôle **62,** le deuxième organe d'alimentation électrique **72** et, le cas échéant, le chargeur magnétique **63,** du dispositif de stimulation **5.** En effet, le dispositif de stimulation **5** peut devoir être réarrangé régulièrement, notamment lorsque le brassard **83** passe d'une porteur à l'autre, ce qui n'est pas le cas du deuxième organe de contrôle **62,** du deuxième organe d'alimentation électrique **72** et, le cas échéant, du chargeur magnétique **63.** Dès lors, la praticité du brassard **83** est améliorée à l'usage.

Le brassard **83** peut comprendre une deuxième portion **832** constituée d'une bande élastique reliant entre elles les deux extrémité circonférentielles de la première portion **831,** la bande élastique étant destiné à être positionnée au niveau de la face interne du membre lorsque le brassard **83** est porté, afin de minimiser les frottements avec le reste du corps, typiquement entre le bras et le buste du porteur, mais aussi afin d'ajuster aisément le brassard **83** sur le moignon **2.**

Le dispositif de stimulation **5** peut comprendre une pluralité d'organes de stimulation **50** positionnés sur le troisième support **83** à distance les uns des autres, en regard du moignon **2.**

Le dispositif de stimulation **5** peut comprendre autant d'organes de stimulation **50** que de premiers supports **81,** c'est-à-dire de préférence trois, chaque organe de stimulation **50** étant configuré pour stimuler le moignon **2** à partir d'une sollicitation mesurée par le (ou les) capteur(s) **40** du premier support **81** correspondant. En d'autres termes, chacun des organes de stimulation **50** est associé à l'un des premiers supports **81.**

Ceci n'est toutefois pas limitatif, puisque le nombre d'organes de stimulation **50** peut être indépendant du nombre de premiers supports **81** et/ou du nombre de capteurs **40.** En tout état de cause, le nombre d'organes de stimulation **50,** et leur position relative sur le moignon **2,** peut être déterminé au cas par cas, en fonction du porteur, notamment par compromis entre la finesse de stimulation, qui peut nécessiter un plus grand nombre d'organes de stimulation **50,** et l'espacement entre les organes de stimulation **50,** pour que le porteur puisse les discriminer et ne pas les confondre. De fait, les terminaisons nerveuses d'un membre amputé ne sont pas les mêmes d'un porteur à l'autre, notamment en fonction du processus de cicatrisation.

Le deuxième organe de contrôle **62** est configuré pour communiquer avec chacun le dispositif de stimulation **5,** de préférence avec chacun des organes de stimulation **50,** avantageusement par voie filaire **80.** En outre, le deuxième organe d'alimentation électrique **72** est configuré pour fournir une énergie électrique à chacun des organes de stimulation **50,** de préférence par voie filaire **80,** avantageusement au moyen de la même voie filaire **80** que celle permettant au deuxième organe de contrôle **62** de communiquer avec les organes de stimulation **50.** Le cas échéant, les organes de stimulation **50** sont reliés au deuxième organe de contrôle **62,** lequel est relié au deuxième organe d'alimentation électrique **72,** l'énergie électrique issue de ce-dernier à destination des organes de stimulation **50** transitant via le deuxième organe de contrôle **62.**

Le premier organe de contrôle **61** est configuré pour communiquer avec le deuxième organe de contrôle **62,** la communication pouvant être mise en œuvre par voie filaire ou, de préférence, sans fil, typiquement via la version économe en énergie de la technologie Bluetooth appelée « BLE » (pour « Bluetooth Low Energy » dans la terminologie anglo-saxonne).

La stimulation du moignon **2** mise en œuvre par le dispositif de stimulation **5** à partir de sollicitations exercées sur la prothèse **1** mesurés par le dispositif de mesure **4** peut être mise en œuvre selon différentes modalités. Typiquement, l'intensité de la stimulation peut être corrélée positivement à l'intensité de la sollicitation mesurée. En outre, le dispositif de contrôle **61, 62** peut comprendre une mémoire dans laquelle sont enregistrés des programmes prédéterminés reliant mesure de la sollicitation et stimulation. Par exemple, si une pression sur un doigt de la prothèse **1** est mesurée durant plus d'une durée donnée, typiquement cinq secondes, le dispositif de stimulation **5** peut générer une vibration sur le moignon **2** par salves périodiques, et non continues, afin de ne pas procéder à une stimulation entêtante pour le porteur pendant une préhension longue, typiquement le port d'un objet.

## Revendications

1. Système haptique (3) pour une prothèse (1) fixée à un moignon (2), le système comprenant :
un capteur (40) ;
un manchon (81) configuré pour être porté sur un doigt de la prothèse (1) et comprenant :
une première couche (811) destinée à être au contact du doigt lorsque le manchon (81) est porté sur le doigt, la première couche (811) étant formée en un premier matériau présentant une première dureté Shore ;
une deuxième couche (812) en contact avec la première couche (811), le capteur (40) étant noyé dans la deuxième couche (812), la deuxième couche (812) étant formée en un deuxième matériau, distinct du premier matériau, et présentant une deuxième dureté Shore strictement supérieure à la première dureté Shore ; et
une troisième couche (813) en contact avec la deuxième couche (812) de sorte que la deuxième couche (812) s'étend entre la première couche (811) et la troisième couche (813), la troisième couche (813) étant formée en un troisième matériau, distinct du premier matériau et du deuxième matériau, et présentant une troisième dureté Shore strictement supérieure à la deuxième dureté Shore ; et
un stimulateur (50) configuré pour être fixé sur le moignon (2) et pour stimuler le moignon (2) à partir d'une sollicitation exercée sur la prothèse (1) et mesurée par le capteur (40).

2. Système haptique (3) selon la revendication 1, dans lequel le manchon (81) est constitué de la première couche (811), de la deuxième couche (812), et de la troisième couche (813).

3. Système haptique (3) selon l'une quelconque des revendications 1 et 2, dans lequel la première dureté Shore est supérieure ou égale à Shore 00-10 et inférieure ou égale à Shore 00-50, la deuxième dureté Shore est supérieure ou égale à Shore 5A et inférieure ou égale à Shore 15A, et/ou la troisième dureté Shore est supérieure ou égale à Shore 25A et inférieure ou égale à Shore 35A.

4. Système haptique (3) selon l'une quelconque des revendications 1 à 3, dans lequel le premier matériau présente une première élongation à la rupture, le deuxième matériau présente une deuxième élongation à la rupture, et le troisième matériau présente une troisième élongation à la rupture ;
dans lequel la première élongation à la rupture est strictement supérieure à la deuxième élongation à la rupture, et la deuxième élongation à la rupture est strictement supérieure à la troisième élongation à la rupture.

5. Système haptique (3) selon la revendication 4, dans lequel la première élongation à la rupture est supérieure ou égale à 800 % et inférieure ou égale à 1000 %, la deuxième élongation à la rupture est supérieure ou égale à 550 % et inférieure ou égale à 750 %, et/ou la troisième élongation à la rupture est supérieure ou égale à 250 % et inférieure ou égale à 450 %.

6. Système haptique (3) selon l'une quelconque des revendications 1 à 5, dans lequel la première couche (811) présente une première épaisseur, la deuxième couche (812) présente une deuxième épaisseur, et la troisième couche (813) présente une troisième épaisseur ;
dans lequel chacune de la première épaisseur, de la deuxième épaisseur, et de la troisième épaisseur, est supérieure ou égale à 0,7 mm et inférieure ou égale à 1,5 mm.

7. Système haptique (3) selon la revendication 6, dans lequel la première épaisseur, la deuxième épaisseur, et la troisième épaisseur sont identiques entre elles.

8. Système haptique (3) selon l'une quelconque des revendications 1 à 7, dans lequel chacun du premier matériau, du deuxième matériau, et du troisième matériau, est un silicone.

9. Système haptique (3) selon l'une quelconque des revendications 1 à 8, comprenant en outre :
un support (82) configuré pour être fixé à la prothèse (1) de manière amovible ; et
un organe de contrôle (61) fixé sur le support (82), et configuré pour être relié via un fil électrique au capteur (40) pour en recevoir un signal représentatif d'une mesure de la sollicitation exercée sur la prothèse (1).

10. Système haptique (3) selon l'une quelconque des revendications 1 à 9, dans lequel :
le premier matériau est obtenu par une première polymérisation mise en œuvre pendant une première durée supérieure ou égale à 3 minutes et inférieure ou égale à 15 minutes ;
le deuxième matériau est obtenu par une deuxième polymérisation mise en œuvre pendant une deuxième durée supérieure ou égale à 30 minutes et inférieure ou égale à 120 minutes ; et
le troisième matériau est obtenu par une troisième polymérisation mise en œuvre pendant une troisième durée supérieure ou égale à 600 minutes et inférieure ou égale à 1200 minutes.
